# EUROPEAN PATENT APPLICATION

(11) **EP 1 080 710 A2**
(43) Date of publication of application: **07.03.2001**
(21) Application number: 00113513.6
(22) Date of filing: 26.06.2000
(51) Int. Cl.: A61G 7/07

(54) **Holding device for fastening a support cushion to one's back**

(30) Priority: 30.08.1999 JP 24391999
(71) Applicant: Hiroshi, Kamma, Tokyo (JP)
(72) Inventor: Hiroshi, Kamma, Tokyo (JP)
(74) Representative: Hartz, Nikolai F., Dr.

(57) **Abstract**

A tool for firmly carrying a cushiony member on a user's back includes a coupler to be coupled to a cushiony member and a shoulder band. The coupler is supposed to be coupled to the cushiony member. The cushiony member is supposed to be positioned on a user's back and located between the back and a bed to work for keeping him or her lying on his or her side when he or she lies on the bed and leans against the cushiony member. The shoulder band is coupled to the coupler, allowing him or her to carrying the cushiony member coupled to the coupler on the user's back.

## Description

The present invention relates to a tool for firmly carrying a cushiony member on one's back, which may be used to relieve a bedsore, a snore and so on, in particular.

A bedridden person can have bedsores easily since the circulation of the blood degrades at a pressed portion of his or her body. In order to avoid the bedsores, he or she has been made to change his or her position on a bed, or a doughnut-shaped pillow has been put under his or her buttocks. If a snoring person continuously lies on his or her back, his or her base of the tongue lowers to make the snore terrible, which causes lack of sleep or a sleep apnea syndrome.

There have been created problems in that it takes a lot of labor to periodically change his or her position on a bed or to put a doughnut-shaped pillow under his or her buttocks as before, and that such measures could become unreliable by his or her body movement and so on.

It is an object of the present invention to eliminate these problems, and to provide a tool enabling person to leaning his or her body against a cushiony member firmly carried on his or her back to naturally lie on his or her side, preventing him or her from having bedsores or easily snoring.

It is another object of the present invention to provide a tool capable of using a shoulder band to fix the cushiony member on the body of a patient or a bedridden person, making the cushiony member followed his or her body movement.

It is another object of the present invention to provide a tool for firmly carrying a cushiony member on one's back, which has a simple structure.

It is another object of the present invention to provide a tool capable of winding a band around the cushiony member to fix the cushiony member to the shoulder bands.

It is another object of the present invention to provide a tool capable of housing a cushiony member in a bag to fix the cushiony member to the shoulder bands.

It is another object of the present invention to provide a tool capable of using a waist belt to put the cushiony member on the body in close contact.

It is another object of the present invention to provide a tool capable of using a breast belt to put the cushiony member on the body in close contact.

In a first mode of the present invention, the tool for firmly carrying a cushiony member on a user's back includes a coupler to be coupled to a cushiony member and a shoulder band. The coupler is supposed to be coupled to the cushiony member. The cushiony member is supposed to be positioned on a user's back and located between the back and a bed to work for keeping him or her lying on his or her side when he or she lies on the bed and leans against the cushiony member. The shoulder band is coupled to the coupler, allowing him or her to carrying the cushiony member coupled to the coupler on the user's back.

In a second mode of the present invention, the cushiony member may be fixed to the coupler.

In a third mode of the present invention, the coupler may be a winding band. The winding band may be wound around the cushiony member.

In a fourth mode of the present invention, the coupler may be a bag. The bag is served to house the cushiony member.

In a fifth mode of the present invention, the shoulder band may have a waist belt coupled thereto.

In a sixth mode of the present invention, the shoulder band may have a breast belt coupled thereto.

In accordance with the present invention, the following advantages are offered:

In the first mode, the user can lean his or her body against the cushiony member firmly carried on the back to naturally lie on his or her side, preventing him or her from having bedsores or easily snoring. This mode can eliminate the cause of lack of sleep or a sleep apnea syndrome. This mode can also use the shoulder band to fix the cushiony member to the user's body, causing the cushiony member to follow his or her body movement even if he or she is a patient or a bedridden person.

In the second mode, the tool can offer an advantage to provide a simple structure in addition to the advantages offered by the first mode.

In the third mode, the user can offer an advantage to wind the winding band around the cushiony member to fix the cushiony member to the shoulder band in addition to the advantages offered by the first mode. As a result, the tool is easy to carry before the tool has had the cushiony member combined therewith.

In the fourth mode, the user can offer an advantage to house the cushiony member and fix the cushiony member to the shoulder band in addition to the advantages offered by the first mode. As a result, the tool is easy to carry before the tool has had the cushiony member combined therewith.

In the fifth mode, the user can offer an advantage to use the waist belt to put the cushiony member on his or her body in close contact in addition to the advantages offered by the first mode. As a result, the user can have an improved feeling of fit to the cushiony member, decreasing a feeling of discomfort caused by wearing.

In the sixth mode, the user can offer an advantage to use the breast belt to put the cushiony member on his or her body in close contact in addition to the advantages offered by the first mode. As a result, the user can have an improved feeling of fit to the cushiony member, decreasing a feeling of discomfort caused by wearing.

The invention will now be described by way of example and with reference to the accompanying drawings in which:
Figure 1 is a side view of an example of the tool for firmly carrying a cushiony member on a user's back according to a first embodiment of the present invention, showing how to use the tool;
Figure 2 is a perspective view of the example;
Figure 3 is a perspective view of the example, showing how to use the tool;
Figure 4 is a perspective view of an example of the tool for firmly carrying a cushiony member on a user's back according to a second embodiment of the present invention; and
Figure 5 is a perspective view of an example of the tool for firmly carrying a cushiony member on a user's back according to a third embodiment of the present invention.

### FIRST ENBODIMENT

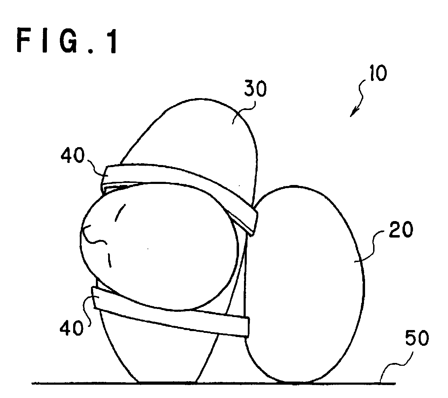

In the drawings, the first numeral 10 designates a tool for firmly carrying a cushiony member on a user's back. The tool 10 is served to carry a cushiony member 20 on a user's back 30 as shown in Figures 1-3. The tool 20 basically includes the following parts as shown in Figures 1-3.

### (1) The cushiony member 20

### (2) Shoulder bands 40

The tool 10 is not limited to one with the parts (1) and (2) mounted thereto, and may include an additional part. When a user lies on a bed 50, carrying the cushiony member 20 on the back, and leans against the cushiony member 20, the cushiony member is positioned on the user's back 30 and located between the back and the bed 50 to work for keeping him or her lain on his or her side as shown in Figures 1-3. The cushiony member 20 is a bag with a suitable amount of sponge filled therein, for instance, though not shown.

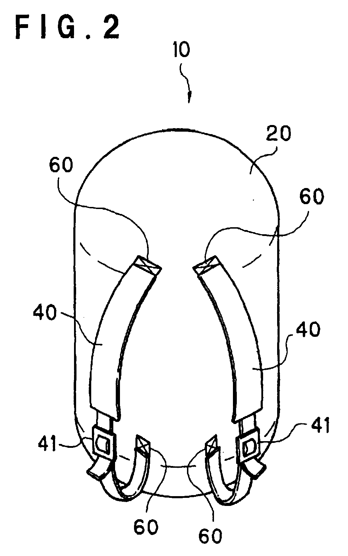

The user can use the shoulder bands 40 to carry the cushiony member 20 on his or her back. The shoulder bands are formed in a belt shape and are positioned at two locations, i.e., on right and left positions of his or her body as in Figures 1-3. As shown in Figure 2, buckles 41 are mounted to the shoulder bands 40 to provide the shoulder bands with adjustable length. In place of the buckles 41, the shoulder bands 40 may be made of a rubber material to have adjustable length. The number of the shoulder bands 40 is not limited to two but may be one. The shoulder bands 40 are not limited to ones in a belt shape but may be clothing, such as a vest and night clothing.

Both ends of each of the right and left shoulder bands 40 are fixed to the cushiony member 20 through couplers 60 as shown in Figure 2. The couplers 60 may be provided by sewing totally four of the respective ends of the right and left shoulder bands 40 on the cushiony member 20 as shown in Figure 2. Instead of sewing the respective ends of the shoulder bands 40 on the cushiony member 20, the shoulder bands may be coupled to the cushiony member 20 through rings.

The tool 10 thus constructed will be used as follows:

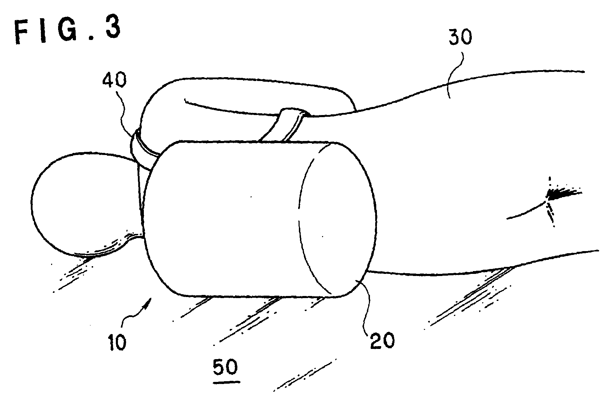

Specifically, the user slings the two shoulder bands 40 over his or her shoulders to carry the cushiony member 20 on the back as shown in Figures 1 and 3. As shown in Figures 1 and 3, the user lies down on the bed 50, carrying the cushiony member 20 on the back. When the user leans against the cushiony member 20, lying down, the cushiony member 20 is crushed and deformed between his or her body 30 and the bed 50 as shown in Figures 1 and 3.

As the result of the deformation, the cushiony member 20 creates an elastic restoring force to push the back of his or her body 30 back, and the user can easily maintain a posture to lie on his or her side. Even if the user applies the weight of his or her body to the cushiony member 20, one of his or her shoulders is forced to rise away from the bed 50 to prevent the user from lying on the back since the cushiony member 20 exists between the bed 50 and his or her body.

### SECOND EMBODIMENT

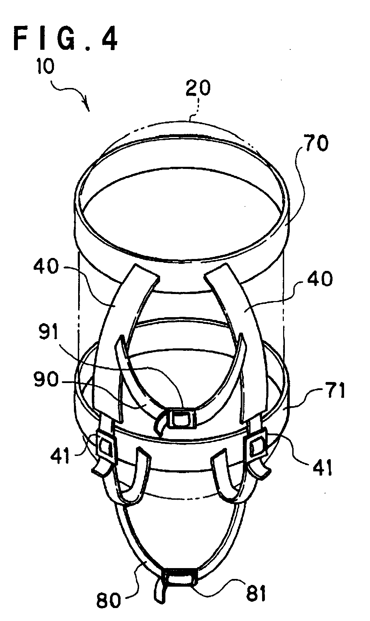

Now, referring to Figure 4, explanation of a second embodiment of the tool 10 will be made.

This embodiment is characterized in that the couplers are constituted by bands 70 and 71 as shown in Figure 40. Specifically, totally two bands 70 and 71 are provided at upper and lower positions as shown in Figure 4. The upper band 70 is fixedly sewed on the respective upper ends of the right and left shoulder bands 40. The lower band 71 is fixedly sewed on the respective lower ends of the right and left shoulder bands 40. The upper and lower bands 70 and 71 may have adjustable length or be extensive and retractable according to the outer diameter of the cushiony member 20, though not shown. The number of the bands 70 and 71 is not limited to two, but the number may be one or not less than three. When the upper and lower bands are used, the bands are fixedly wound around a cushion, a pillow or a similar one, though not shown.

In accordance with this embodiment, a cushion or pillow may be utilized as the cushiony member 20. The tool is easy to carry before the tool has had the cushiony member 20 combined therewith.

The right and left shoulder bands 40 include a waist belt 80 and a breast belt 90 as shown in Figure 4. The waist belt 80 and the breast belt 90 have adjustable length since both belts include buckles 81 and 91 as shown in Figure 4. Instead of using the buckle 81 or 91, the waist belt 80 or the breast belt 90 may be made of a flexible material, such as rubber, so as to have adjustable length. The waist belt 80 or the breast belt 90 can put the cushiony member on the user's body 30 in closer contact, relieving a feeling of discomfort.

### THIRD EMBODIMENT

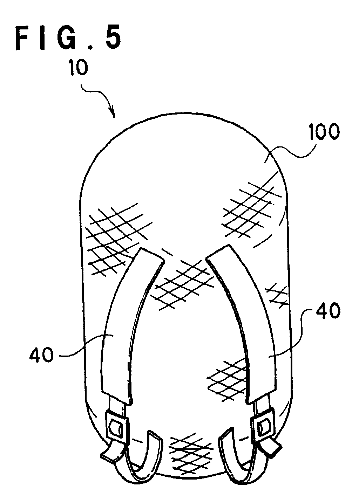

Referring now to Figure 5, explanation of the tool 10 according to a third embodiment will be explained.

This embodiment is characterized in that the coupler is a bag 100 as shown in Figure 5. Specifically, the bag 100 has a network structure as shown in Figure 5. The bag 100 has a portion, such as an upper side and a lateral side, formed to be openable, though not shown. The bag 100 is not limited to have a network structure as long as the bag is formed in an envelope shape. When the bag 100 is used, a cushion, a pillow, a collection of sponge pieces, a collection of pieces of cloth or another cushiony member is put into the bag through the opening (not shown) and the bag is closed.

According to this embodiment, various kinds of cushiony members can be utilized as the cushiony member 20 by being put into the bag 100. The tool 10 is easy to carry before the tool has had the cushiony member 20 combined therewith.

Obviously, numerous modifications and variations of the present invention are possible in light of the above teachings. It is therefore to be understood that within the scope of the appended claims, the invention may be practiced otherwise than as specifically described herein.

The entire disclosure of Japanese Patent Application No. 11-243919 filed on August 30, 1999 including specification, claims, drawings and summary are incorporated herein by reference in its entirety.

## Claims

1. A tool for firmly carrying a cushiony member on a user's back, comprising:
a coupler (60) to be coupled to a cushiony member (20), the cushiony member being positioned on a user's back and located between the back and a bed (50) to keep him or her lying on his or her side when he or she lies on the bed and leans against the cushiony member; and
a shoulder band (40) for carrying the cushiony member on the user's back, the shoulder band coupled to the coupler.

2. The tool according to Claim 1, wherein the coupler (60) has the cushiony member (20) fixed thereto.

3. The tool according to Claim 1, wherein the coupler (60) comprises a winding band (70 or 71) to be wound around the cushiony member (20).

4. The tool according to Claim 1, wherein the coupler (60) comprises a bag (100) for housing the cushiony member (20).

5. The tool according to Claim 1, wherein the shoulder band (40) has a waist belt (80) coupled thereto.

6. The tool according to Claim 1, wherein the shoulder band (40) has a breast belt (90) coupled thereto.
